# EUROPÄISCHE PATENTANMELDUNG

(11) **EP 1 454 933 A1**
(43) Veröffentlichungstag der Anmeldung: **08.09.2004**
(21) Anmeldenummer: 03104606.3
(22) Anmeldetag: 09.12.2003
(51) Int. Cl.: C08G 18/02, C07D 251/34

(54) **Verfahren zur Herstellung von geruchsarmen und lagerstabilen monomerhaltigen Polyisocyanuraten auf Basis von Isophorondiisocyanat**

(30) Priorität: 05.03.2003 DE 10309432
(71) Anmelder: Degussa AG, 40474 Düsseldorf (DE)
(72) Erfinder: Kohlstruk, Stephan, Dr, 48249 Dülmen (DE); Kreczinski, Manfred, 44652 Herne (DE); Elm, Rainer, Dr., 45772 Marl (DE)

(57) **Zusammenfassung**

Verfahren zur Herstellung von geruchsarmen und lagerstabilen monomerhaltigen Polyisocyanuraten aus Isophorondiisocyanat, wobei die partielle Trimerisierung innerhalb von 2 Minuten bis 30 Minuten erfolgt in Gegenwart von 0,05 bis 1,5 Gew.-%, basierend auf dem Gewicht des Diisocyanats, eines Katalysators der allgemeinen Formel

[R-NX₃]^{m⊕}•mY^{⊖}

in der Y⁻ für ein Carbonsäureanion mit 4 bis 8 C-Atomen und R für eine β-Hydroxyalkylgruppe mit 2 bis 6 C-Atomen steht und X eine Alkylengruppe mit 2 bis 3 C-Atomen darstellt, wobei die drei Reste X über ein gemeinsames, ggf. partiell β-hydroxyalkyliertes Stickstoffatom mit dem quarternären Stickstoff einen tricyclischen Ring bilden, der ggf. über eine zum Stickstoff α- oder β- oder γ-ständige OH-Gruppe verfügt und m eine Zahl von 1,0 bis 2,0 bedeutet, dadurch gekennzeichnet, dass die Trimerisierung kontinuierlich in einem Rohrreaktor, insbesondere in einer Reaktionsschlange, in einem Temperaturbereich von 20-120°C und einem Druckbereich von 0,5 bis 5 bar durchgeführt und der Katalysator im Anschluss bei 100-160°C thermisch deaktiviert wird.

## Beschreibung

Die vorliegende Erfmdung betrifft ein Verfahren zur Herstellung von geruchsarmen und lagerstabilen monomerhaltigen Polyisocyanuraten auf Basis von Isophorondiisocyanat.

Polyisocyanurate sind als Polyisocyanataddukte wertvolle Komponenten zur Herstellung von hochwertigen Beschichtungen mit guten mechanischen Eigenschaften sowie guter Licht- und Wetterbeständigkeit. Polyisocyanurate auf Basis von Isophorondiisocyanat (IPDI) finden zudem Einsatz als Rohstoff für PUR-basierende Elastomeranwendungen. Dabei kann es wünschenswert sein, dass das IPDI-basierende Polyisocyanurat, man spricht auch von IPDI-Trimer, in monomerhaltiger Form eingesetzt wird.

Grundsätzlich werden Polyisocyanurate durch katalytische Trimersierung geeigneter Isocyanate erhalten. Geeignete Isocyanate sind z. B. aromatische, cycloaliphatische und aliphatische di- und höherwertige Polyisocyanate. Als Katalysatoren kommen z. B. tert. Amine (US 3,996,223), Alkalimetallsalze von Carbonsäuren (CA 2113890; EP 56159), quart. Ammoniumsalze (EP 798299; EP 524501; US 4,186,255; US 5,258,482; US 4,503,226; US 5,221,743), Aminosilane (EP 197864; US 4,697,014) und quart. Hydroxyalkylammoniumsalze (EP 17998; US 4,324,879) in Frage. In Abhängigkeit vom Katalysator ist auch die Verwendung von diversen Co-Katalysatoren möglich, z. B. OH-funktionalisierte Verbindungen oder Mannich Basen aus sek. Aminen und Aldehyden bzw. Ketonen.

Zur Trimerisierung lässt man die Polyisocyanate in Gegenwart des Katalysators, ggf. unter Verwendung von Lösemitteln und/oder Hilfsstoffen, bis zum Erreichen des gewünschten Umsatzes reagieren. Man spricht in diesem Zusammenhang auch von partieller Trimerisierung, da der angestrebte Umsatz in der Regel deutlich unterhalb von 100 % liegt. Danach wird die Reaktion durch Deaktivierung des Katalysators abgebrochen. Dies erfolgt durch Zusatz eines Katalysatorinhibitors wie beispielsweise p-Toluolsulfonsäure, Chlorwasserstoff oder Dibutylphosphat und hat zwangsläufig eine ggf. unerwünschte Kontamination des entstandenen isocyanuratgruppenhaltigen Polyisocyanats zur Folge. Besonders vorteilhaft im Hinblick auf die Trimerisierung von Isocyanaten im technischen Maßstab ist der Einsatz von quart. Hydroxyalkylammoniumcarboxylaten als Oligomerisierungskatalysatoren. Dieser Katalysatortyp ist thermisch labil und erlauben eine gezielte thermische Deaktivierung, so dass es unnötig ist, die Trimerisierung bei Erreichen des gewünschten Umsatzes durch Zudosierung potentiell qualitätsmindernder Inhibitoren abzustoppen.

Monomerhaltiges IPDI-Trimer, welches beispielsweise für o. g. PUR-Spritzapplikationen geeignet ist, weist einen NCO-Gehalt von 28 bis 32 Gew.-% auf. Das Polyisocyanurat wird durch partielle Trimerisierung von IPDI in Gegenwart eines oder mehrerer geeigneter Katalysatoren hergestellt. Danach muss der Katalysator aus der Reaktionslösung entweder vollständig entfernt - dies kann durch Kurzweg- oder Dünnschichtdestillation erfolgen - oder deaktiviert werden, weil das Trimerisat in Gegenwart aktiver Katalysatorrückstande nicht lagerstabil ist. Liegt der NCO-Gehalt des erhaltenen IPDI-Polyisocyanurats unterhalb des gewünschten Niveaus, kann er durch Verdünnung der Lösung mit monomerem IPDI problemlos wunschgemäß eingestellt werden.

Alkalimetallsalze von Carbonsäuren sind als Katalysatoren für die Herstellung von monomerhaltigem IPDI-Trimer nicht gut geeignet, da sie sich nur schwierig oder gar nicht aus dem Reaktionsprodukt entfernen lassen. In bezug auf die verfügbaren aminhaltigen Katalysatoren hat es sich erwiesen, dass die resultierenden IPDI-Trimer-Lösungen grundsätzlich mit einem deutlich wahrnehmbaren Geruch behaftet sind, der ausgeprägt genug ist, auch in der Endapplikation noch spürbar und unangenehm in Erscheinung zu treten. Zur Beseitigung der Geruchsbelästigung wird die Reaktionslösung in der technischen Praxis nach partieller Trimerisierung und Katalysatordeaktivierung vom überschüssigen IPDI, von geruchsgebenden Komponenten und ggf. von unerwünschten Katalysatoreninhibitoren befreit. Dies geschieht in der Regel durch Kurzweg- oder Dünnschichtdestillation. Im Anschluss wird das monomerbefreite Festharz unter Zudosierung von frischem IPDI in das gewünschte, geruchsarme und monomerhaltige IPDI-Polyisocyanurat überführt.

Die Sequenz aus partieller Trimerisierung/Deaktivierung, Entmonomerisierung/ Reinigung und abschließender Lösung des Festharzes im Monomer ist sehr aufwendig. Zeit- und kostentreibender Schritt und zudem kapazitätslimitierender Engpass des bestehenden Verfahrens ist vor allem der Schritt der Monomerabtrennung. Ein wirtschaftlicheres Verfahren zur Herstellung von geruchsarmen und lagerstabilen monomerhaltigen Polyisocyanuraten auf Basis von Isophorondiisocyanat hätte vor allem einen Verzicht auf die Entmonomerisierung zur Voraussetzung.

Aus der EP 1 273 603 ist bekannt, dass die Entmonomerisierung eingespart werden und zudem der Einsatz von ggf. qualitätsmindernden Inhibitoren vermieden werden kann, wenn die Trimerisierung von IPDI im Batchverfahren oder auch kontinuierlich (Kesselkaskade oder Kombination aus Kesselkaskade und Rohrreaktor) in Gegenwart spezieller Katalysatoren durchgeführt wird. Nachteil des Verfahrens ist jedoch der relativ hohe Katalysatorbedarf und die damit verbundenen Kosten für den Katalysator. Zudem liegt die Reaktivität des Endprodukts des bekannten Verfahrens gegenüber OH-funktionellen Reaktionspartnern, die bei der Herstellung von PUR-Beschichtungen und PUR-basierenden Elastomeren zum Einsatz gelangen, auf hohem Niveau. Eine verringerte Reaktivität des monomerhaltigen Polyisocyanurats wäre hier wünschenswert, da sie dem Verarbeiter einen größeren Spielraum im Hinblick auf die optimale Anpassung des Verarbeitungszeitraums auf die Bedürfnisse der jeweiligen Applikation gestattet.

Es war daher Aufgabe der vorliegenden Erfindung, ein kontinuierliches Verfahren zur Herstellung von IPDI-basierenden geruchsarmen und lagerstabilen monomerhaltigen Polyisocyanuraten zu entwickeln, welches nicht die genannten Nachteile des Stands der Technik aufweist.

Gegenstand der Erfmdung ist deshalb ein Verfahren zur Herstellung von geruchsarmen und lagerstabilen monomerhaltigen Polyisocyanuraten aus Isophorondiisocyanat, wobei die partielle Trimerisierung innerhalb von 2 Minuten bis 30 Minuten erfolgt in Gegenwart von 0,05 - 1,5 Gew.-%, basierend auf dem Gewicht des Diisocyanats, eines Katalysators der allgemeinen Formel

[R-NX₃]^{m⊕} • mY^{⊖}

in der Y⁻ für ein Carbonsäureanion mit 4 bis 8 C-Atomen und R für eine β-Hydroxyalkylgruppe mit 2 - 6 C-Atomen steht und X eine Alkylengruppe mit 2 bis 3 C-Atomen darstellt, wobei die drei Reste X über ein gemeinsames, ggf. partiell β-hydroxyalkyliertes Stickstoffsatom mit dem quartemären Stickstoff einen tricyclischen Ring bilden, der ggf. über eine zum Stickstoff α-, β- oder γ-ständige OH-Gruppe verfügt und m eine Zahl von 1,0 bis 2,0 bedeutet, dadurch gekennzeichnet, dass die Trimerisierung kontinuierlich in einem Rohrreaktor, insbesondere in einer Reaktionsschlange, in einem Temperaturbereich von 20 bis 120 °C, vorzugsweise 40 bis 110 °C, besonders bevorzugt 55 bis 95 °C und einem Druckbereich von 0,5 bis 5 bar, vorzugsweise 0,5 bis 2 bar, besonders bevorzugt 0,9 bis 1,5 bar, durchgeführt und der Katalysator im Anschluss bei 100-160°C thermisch deaktiviert wird. Dabei kann auf eine Monomerabtrennung verzichtet werden.

Grundsätzlich können zur Trimerisierung geeignete Isocyanate nach verschiedenartigen Verfahren hergestellt werden (Annalen der Chemie 562 (1949), S. 75ff.). Technisch insbesondere bewährt hat sich die Herstellung durch Phosgenierung von organischen Polyaminen zu den entsprechenden Polycarbaminsäurechloriden und deren thermische Spaltung in organische Polyisocyanate und Chlorwasserstoff. Alternativ können organische Polyisocyanate auch ohne die Verwendung von Phosgen, d. h. nach phosgenfreien Verfahren, hergestellt werden. Nach Angaben der EP-A-126 299 (USP 4,596,678), EP-A-126 300 (USP 4,596,679) und EP-A-355 443 (USP 5,087,739) beispielsweise können (cyclo)aliphatische Diisocyanate wie 1-Isocyanato-3-isocyanatomethyl-3,5,5-trimethylcyclohexan (Isophorondiisocyanat bzw. IPDI) zugänglich gemacht werden durch Umsetzung der zugrundeliegenden (cyclo)aliphatischen Diamine mit Harnstoff und Alkoholen zu (cyclo)aliphatischen Biscarbaminsäureestern und deren thermische Spaltung in die entsprechenden Diisocyanate und Alkohole.

Für das erfindungsgemäße Verfahren zur Herstellung von geruchsarmen und lagerstabilen monomerhaltigen Polyisocyanuraten auf Basis von Isophorondiisocyanat ist es unwesentlich, über welchen Syntheseweg das eingesetzte IPDI hergestellt wurde. Es sei jedoch darauf hingewiesen, dass die zur Erreichung eines wunschgemäßen NCO-Gehaltes notwendige Katalysatormenge auch von der Qualität des Rohstoffes abhängt. Erfahrungsgemäß macht ein ansteigender Gehalt des IPDI an hydrolysierbaren Chlorverbindungen grundsätzlich eine Erhöhung der Katalysatormenge erforderlich. Anscheinend übt das hydrolysierbare Chlor tendenziell einen inhibitiven Effekt auf den Kontakt aus.

Die erfindungsgemäße Herstellung der geruchsarmen und lagerstabilen monomerhaltigen Polyisocyanuraten auf Basis von Isophorondiisocyanat durch partielle Trimerisierung erfolgt kontinuierlich in einem Rohrreaktor, insbesondere in einer Reaktionsschlange. Der Katalysator wird in geringer Konzentration zwischen 0,05 und 1,5 Gew.-% eingesetzt. Die exakte Menge lässt sich leicht experimentell ermitteln und ist abhängig vom Katalysator, vom Umsatzziel sowie von der Qualität des eingesetzten IPDI.

Die partielle Trimerisierung lässt sich innerhalb von 2 Minuten bis 30 Minuten durchführen. Das Produkt enthält monomeres IPDI, trimeres IPDI-Isocyanurat und höhere Oligomere mit Isocyanuratstruktur. Als Nebenkomponente können in geringer Menge ggf. auch Verbindungen mit Uretdionstruktur gefunden werden. Verbindungen dieser Art sind in der Literatur beschrieben.

Erfindungsgemäß wird der Katalysator in einer Menge von 0,05 - 1,5 Gew.-%, basierend auf dem Gewicht des eingesetzten Isophorondiisocyanats, eingesetzt. Der Katalysator lässt sich leicht durch Reaktion der drei Grundbausteine - tricyclisches Diamin, Carbonsäure und Oxiran - erhalten. Die Herstellung kann ggf. in Gegenwart eines Lösemittels erfolgen. Üblicherweise kommen niedermolekulare Alkohole wie Methanol oder Ethylenglykol zum Einsatz. Als tricyclisches Diamin kommt z. B. Diazabicyclo[2.2.2]octan in Frage. Geeignete Carbonsäuren sind z. B. Essigsäure, Hexansäure oder 2-Ethylhexansäure. Mögliche Optionen für die Oxirankomponente sind z. B. Propylenoxid, Butylenoxid oder 1,2-Epoxyhexan. Die Molverhältnisse der drei Grundbausteine zur Herstellung der erfmdungsgemäß eingesetzten Katalysatoren sind variabel. Je nach Verhältnis der Grundbausteine werden Katalysatoren mit mindestens einem quaternären Stickstoffatom im Molekül erhalten. Wie die Beispiele zeigen, werden auch solche Katalysatoren eingesetzt, die teilweise über zwei quaternäre Stickstoffatome verfügen.

Das erfmdungsgemäße Verfahren wird bei Temperaturen zwischen 20 °C und 120 °C, vorzugsweise zwischen 40 °C und 110 °C, und besonders bevorzugt zwischen 55 und 95 °C durchgeführt.

Erfindungsgemäß wird die partielle Trimerisierung des Isophorondiisocyanats kontinuierlich in einem Rohrreaktor, in der bevorzugten Ausführungsform in einer Reaktionsschlange, durchgeführt.

Zwei Stoffströme, Isophorondiisocyanat und der entsprechende Katalysator, werden kontinuierlich durch einen mechanischen Mischer gefahren. In dem Mischer wird der Katalyator bei Temperaturen zwischen 20 °C und 40 °C im Isophorondiisocyanat homogenisiert. Anschließend durchströmt das Gemisch einen Rohrreaktor, in der bevorzugten Ausführungsform eine Reaktionsschlange, in dem die Trimerisierung bei Temperaturen zwischen 20 °C und 120 °C, vorzugsweise zwischen 55 °C und 95 °C, erfolgt. Der Rohrreaktor, in der bevorzugten Ausführungsform eine Reaktionsschlange, hat mehrere temperierbare Zonen. Diese erlauben jederzeit eine optimale Prozesskontrolle sowohl in bezug auf den Umsatz als auch hinsichtlich des Temperaturprofils der Reaktion. Nach erreichen des gewünschten Umsatzes wird das Reaktionsgemisch bei 100 bis 160 °C thermisch gequentscht, wobei der Katalysator zerstört wird. Das so erhaltene Reaktionsgemisch ist lagerstabil und geruchsarm.

Der Katalysator kann in reiner Form eingesetzt werden. Zur exakteren Dosierung und optimalen Durchmischung des Katalysators kann es jedoch vorteilhaft sein, den Katalysator in einem geeignetem Lösemittel zu lösen. Geeignet sind prinzipiell solche Lösemittel, in denen der Katalysator eine gute Löslichkeit hat, z. B. Wasser, niedermolekulare Alkohole wie Methanol oder Ethylenglykol oder niedermolekulare organische Säuren wie beispielsweise Essigsäure oder Hexansäure.

Die erfindungsgemäß hergestellten geruchsarmen und lagerstabilen monomerhaltigen Polyisocyanuraten auf Basis von Isophorondiisocyanat stellen nützliche Zwischenprodukte für Polyurethanbeschichtungen, Polyurethan- und Polyharnstoff-Formteile wie sie beispielsweise im Rahmen des RIM-Verfahrens ("Reaction Injection Moulding") produziert werden, für Polyurethan-Spritzapplikationen oder auch für Polyurethan-basierenden Automobilfensterverguss dar. Dabei können sie auch in mit Blockierungsmitteln blockierter Form zum Einsatz gelangen. Geeignete Blockierungsmittel sind dabei beispielsweise Lactame wie ε-Caprolactam, Oxime wie Methylethylketoxim oder Butanonoxim, Triazole wie 1H-1,2,4-Triazol, leicht enolisierbare Verbindungen wie Acetessigester oder Acetylaceton oder auch Malonsäurederivate wie Malonsäurediester.

### Beispiele

### Katalysatorherstellung

### Katalysator 1

70 Gew.-% einer Mischung aus Propylenoxid, 2-Ethylhexansäure und Diazabicyclo[2.2.2]octan (Molverhältnis 1:1:1) wurden in Gegenwart von 30 Gew.-% Ethylenglykol 3 d bei Raumtemperatur gerührt.

### Katalysator 2

60 Gew.-% einer Mischung aus Propylenoxid, 2-Ethylhexansäure und Diazabicyclo[2.2.2]octan (Molverhältnis 1,25:1,25:1,12) wurden in Gegenwart von 40 Gew.-% Ethylenglykol 3 d bei Raumtemperatur gerührt.

### Trimerisierungsexperimente: Beispiele 1 - 2 und Vergleichsbeispiele 1 - 2

Die Umsetzungen erfolgten grundsätzlich unter einer N₂-Atmosphäre.

### Beispiele

### A.1. Trimerisierung von IPDI mit Katalysator 1

Zu einem kontinuierlichen IPDI-Strom von 9000 g/h wurden bei 35 °C 24,0 g/h (0,27 Gew.-%) Katalysator 1 kontinuierlich zudosiert. Die Temperatur in dem Mischer betrug 39 °C. In der nachgeschalteten Reaktionsschlange stellte sich ein Temperaturprofil von 61 °C - 72 °C -86 °C -80 °C ein. Die thermische Deaktivierung der Reaktionslösung erfolgte bei 100 °C. Die so hergestellte Reaktionslösung hatte einen NCO-Gehalt von 29,0 Gew.-%. Man ließ auf Raumtemperatur abkühlen. Der NCO-Gehalt des geruchsarmen Reaktionsproduktes blieb auch bei Lagerung (7 d, 20 bis 30 °C) stabil. Die Verweilzeit in dem Rohrreaktor betrug 135 Sekunden.
Das Reaktionsprodukt wurde mit frischem IPDI auf einen NCO-Gehalt von 29,6 Gew.-% gebracht. Die Gelzeit einer Kombination des Polyisocyanats mit einem OH-Harz (Capa 3050/Oxyester T 1136 7:3, NCO/OH 1:1) wurde zu 85 h bestimmt.

### A.2. Trimerisierung von IPDI mit Katalysator 2

Zu einem kontinuierlichen IPDI-Strom von 9200 g/h wurden bei 39 °C 28,0 g/h (0,30 Gew.-%) Katalysator 2 kontinuierlich zudosiert. Die Temperatur in dem Mischer betrug 42°C. In der nachgeschalteten Reaktionsschlange stellte sich ein Temperaturprofil von 66 °C - 89 °C -89 °C -83 °C ein. Die thermische Deaktivierung der Reaktionslösung erfolgte bei 110 °C. Die so hergestellte Reaktionslösung hatte einen NCO-Gehalt von 28,8 Gew.-%. Man ließ auf Raumtemperatur abkühlen. Der NCO-Gehalt des geruchsarmen Reaktionsproduktes blieb auch bei Lagerung (7 d, 20 bis 30 °C) stabil. Die Verweilzeit in dem Rohrreaktor betrug 130 Sekunden.
Das Reaktionsprodukt wurde mit frischem IPDI auf einen NCO-Gehalt von 29,6 Gew.-% gebracht. Die Gelzeit einer Kombination des Polyisocyanats mit einem OH-Harz (Capa 3050/Oxyester T 1136 7:3, NCO/OH 1:1) wurde zu 83 h bestimmt.

### Vergleichsbeisspiele

### B.1. Trimerisierung von IPDI mit Katalysator 1 (Batchreaktor)

50 kg IPDI wurde in einem Rührkessel vorgelegt und auf 70 °C erwärmt. Anschließend wurden 0,4 Gew.-% Katalysator 1 über einen Zeitraum von ca. 25 min schrittweise so zudosiert, dass die Temperatur der Reaktionslösung nicht über 90 °C anstieg. Die so hergestellte Reaktionslösung hatte einen NCO-Gehalt von 29,1 Gew.-%. Man ließ auf Raumtemperatur abkühlen. Der NCO-Gehalt des geruchsarmen Reaktionsproduktes blieb auch bei Lagerung (7 d, 20 bis 30 °C) stabil.
Das Reaktionsprodukt wurde mit frischem IPDI auf einen NCO-Gehalt von 29,6 Gew.-% gebracht. Die Gelzeit einer Kombination des Polyisocyanats mit einem OH-Harz (Capa 3050/Oxyester T 1136 7 : 3, NCO/OH 1 : 1) wurde zu 72 h bestimmt.

### B.2. Trimerisierung von IPDI mit Katalysator 2 (Batchreaktor)

50 kg IPDI wurde in einem Rührkessel vorgelegt und auf 70 °C erwärmt. Anschließend wurden 0,43 Gew.-% Katalysator 1 über einen Zeitraum von ca. 30 min schrittweise so zudosiert, dass die Temperatur der Reaktionslösung nicht über 90 °C anstieg. Die so hergestellte Reaktionslösung hatte einen NCO-Gehalt von 29,0 Gew.-%. Man ließ auf Raumtemperatur abkühlen. Der NCO-Gehalt des geruchsarmen Reaktionsproduktes blieb auch bei Lagerung (7 d, 20 bis 30 °C) stabil.
Das Reaktionsprodukt wurde mit frischem IPDI auf einen NCO-Gehalt von 29,6 Gew.-% gebracht. Die Gelzeit einer Kombination des Polyisocyanats mit einem OH-Harz (Capa 3050/Oxyester T 1136 7 : 3, NCO/OH 1 : 1) wurde zu 71 h bestimmt.

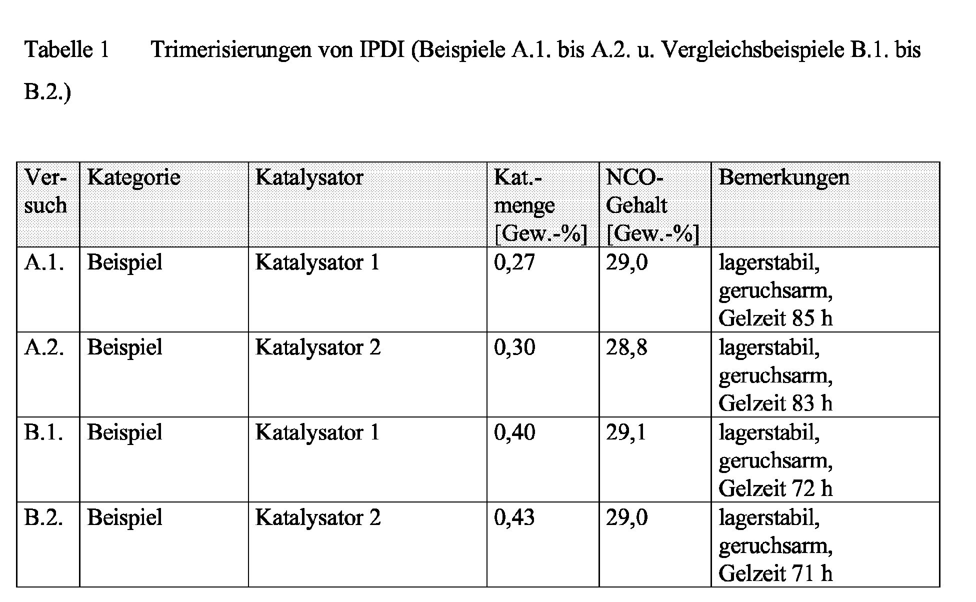

## Patentansprüche

1. Verfahren zur Herstellung von geruchsarmen und lagerstabilen monomerhaltigen Polyisocyanuraten aus Isophorondiisocyanat, wobei die partielle Trimerisierung innerhalb von 2 Minuten bis 30 Minuten erfolgt in Gegenwart von 0,05 - 1,5 Gew.-%, basierend auf dem Gewicht des Diisocyanats, eines Katalysators der allgemeinen Formel
[R-NX₃]^{m⊕} • mY^{⊖}
in der Y⁻ für ein Carbonsäureanion mit 4 - 8 C-Atomen und R für eine β-Hydroxyalkylgruppe mit 2 - 6 C-Atomen steht und X eine Alkylengruppe mit 2 bis 3 C Atomen darstellt, wobei die drei Reste X über ein gemeinsames, ggf. partiell β-hydroxyalkyliertes Stickstoffatom mit dem quarternären Stickstoff einen tricyclischen Ring bilden, der ggf. über eine zum Stickstoff α- oder β- oder γ-ständige OH-Gruppe verfügt und m eine Zahl von 1,0 bis 2,0 bedeutet,
**dadurch gekennzeichnet,**
**dass** die Trimerisierung kontinuierlich in einem Rohrreaktor, insbesondere in einer Reaktionsschlange, in einem Temperaturbereich von 20-120°C und einem Druckbereich von 0,5 bis 5 bar durchgeführt und der Katalysator im Anschluss bei 100-160°C thermisch deaktiviert wird.

2. Verfahren gemäß Anspruch 1,
**dadurch gekennzeichnet,**
**dass** ein Isophorondiisocyanat eingesetzt wird, das nach einem Phosgenverfahren oder einem phosgenfreien Prozess hergestellt wurde.

3. Verfahren nach mindestens einem der vorherigen Ansprüche,
**dadurch gekennzeichnet,**
**dass** das lagerstabile monomerhaltige Polyisocyanurate auf Basis von Isophorondiisocyanat einen NCO-Gehalt von 22 bis 34 Gew.-% aufweist.

4. Verfahren nach mindestens einem der vorherigen Ansprüche,
**dadurch gekennzeichnet,**
**dass** die Umsetzung bei Temperaturen von 40 bis 110 °C erfolgt.

5. Verfahren nach mindestens einem der vorherigen Ansprüche,
**dadurch gekennzeichnet,**
**dass** die Umsetzung bei Temperaturen von 55 bis 95 °C erfolgt.

6. Verfahren nach mindestens einem der vorherigen Ansprüche,
**dadurch gekennzeichnet,**
**dass** die Umsetzung bei einem Druck von 0,5 bis 2 bar erfolgt.

7. Verfahren nach Anspruch 6,
**dadurch gekennzeichnet,**
**dass** die Umsetzung bei einem Druck von 0,9 bis 1,5 bar erfolgt.

8. Verfahren nach mindestens einem der vorherigen Ansprüche,
**dadurch gekennzeichnet,**
**dass** auf eine Monomerabtrennung verzichtet wird.
